# EUROPEAN PATENT SPECIFICATION

(11) **EP 2 688 558 B1**
(45) Date of publication and mention of the grant of the patent: **14.10.2015**
(21) Application number: 12760271.2
(22) Date of filing: 23.03.2012
(51) Int. Cl.: A61K 9/28, A61K 9/48, A61K 47/48, A61K 31/20

(54) **ORAL COMPLEX COMPOSITION COMPRISING OMEGA-3 FATTY ACID ESTER AND HMG-COA REDUCTASE INHIBITOR**
KOMPLEXE ORALE ZUSAMMENSETZUNG AUS EINEM OMEGA-3-FETTSÄURE-ESTER UND EINEM HMG-COA-REDUKTASE-HEMMER
COMPOSITION COMPLEXE ADMINISTRABLE PAR VOIE ORALE CONTENANT DES ESTERS D'ACIDES GRAS OMÉGA-3 ET UN INHIBITEUR DE LA HMG-COA RÉDUCTASE

(30) Priority: 23.03.2011 KR 20110025940; 29.04.2011 KR 20110041168
(43) Date of publication of application: 29.01.2014
(73) Proprietor: Hanmi Pharm. Co., Ltd., Gyeonggi-do 445-910 (KR)
(72) Inventor: KIM, Yong Il, Suwon-si Gyeonggi-do 442-150 (KR); YOON, Eun Jin, Suwon-si Gyeonggi-do 440-710 (KR); IM, Ho Taek, Yongin-si Gyeonggi-do 446-557 (KR); SHIN, Yoon Sub, Suwon-si Gyeonggi-do 440-723 (KR); PARK, Jae Hyun, Suwon-si Gyeonggi-do 443-470 (KR); WOO, Jong Soo, Suwon-si Gyeonggi-do 440-710 (KR)
(74) Representative: Goddar, Heinz J.
(86) International application number: PCT/KR2012/002134
(87) International publication number: WO 2012/128587

(56) References cited:
- EP-A1- 2 229 939
- WO-A2-02/43659
- KR-A- 20090 091 077
- US-A1- 2007 191 467
- US-A1- 2008 089 876
- US-A1- 2009 196 920

## Description

### FIELD OF THE INVENTION

The present invention relates to an oral complex composition comprising omega-3 fatty acid esters and a HMG-CoA reductase inhibitor.

### BACKGROUND OF THE INVENTION

Marine oils, also commonly referred to as fish oils, are the main sources of omega-3 fatty acids, i.e. eicosapentaenoic acid (EPA) and docosahexaenoic acid (DHA), which modulate lipid metabolism. Omega-3 fatty acids can, with no adverse side effects, increase serum high-density lipoprotein (HDL) cholesterol while reducing serum triglycerides (TG), systolic and diastolic blood pressure, the heart rate, and the activation of blood coagulation factor VII-phospholipids complex.

Currently available omega-3 fatty acids drugs are omega-3 fatty acid ethyl esters (hereinafter, referred as "omega-3 fatty acid esters"), an ethyl-esterified concentration of omega-3 fatty acids, i.e. polyunsaturated fatty acids from DHA and EPA-containing fish oils, and is sold under the trademark OMACOR^{®}. Such omega-3 fatty acid esters are generally formulated into a capsule form such as gelatin capsules, as disclosed in U.S. Patent Nos. 5,502,077, 5,656,667 and 5,698,594.

Besides natural fermentation products, mevastatin and lovastatin (MEVACOR^{®}; *see* U.S. Patent No. 4,231,938), different types of synthesized and semisynthesized HMG-CoA reductase inhibitors are exist including simvastatin (ZOCOR^{®}; *see* U.S. Patent No. 4,444,784), pravastatin sodium salt (PRAVACHOL^{®}; *see* U.S. Patent No. 4,346,227), fluvastatin sodium salt (LESCOL^{®}; *see* U.S. Patent No. 5,354,772), atorvastatin calcium salt (LIPITOR^{®}; *see* U.S. Patent No. 5,273,995), cerivastatin sodium salt (also known as rivastatin; *see* U.S. Patent No. 5,177,080), rosuvastatin calcium salt (CRESTOR^{®}; *see* KR. Patent No. 105431) and pitavastatin calcium salt (LIVARO^{®}; *see* KR Patent No. 101149). Such HMG-CoA reductase inhibitors contain 3-hydroxy lactones or corresponding ring opened dihydroxy acids, and are often referred to as "statins." Statins were typically used for treatment to maintain cholesterol levels within the normal range. Statins can inhibit HMG-CoA reductase which regulates cholesterol synthesis thereby slowing down the production of cholesterol, or can reduce serum low-density lipoprotein (LDL) cholesterol by upregulating LDL receptors in the liver. Thus, the main function of the statins is diminishing LDL cholesterol. Statins are known to reduce the risk of coronary heart disease (CHD) by one third, yet have limited effects on TG and serum HDL.

Patients with hypercholesterolemia and mixed dyslipidemia show high levels of LDL and TG. It is advantageous to use a pharmaceutical combination of omega-3 fatty acid esters and statins because it is applicable to treat high levels of both LDL and TG.

Therefore, development of a pharmaceutical combination of omega-3 fatty acid esters and statins may be useful for treating hyperlipidemia by raising serum HDL while reducing LDL and TG levels. Hence, there has been much research on combined formulation of omega-3 fatty acid esters. For example, U.S. Patent Publication No. 2007/0212411 disclosed combined formulations of OMACOR^{®} by conducting, in sequence, polymer barrier coating, drug coating and top coating. Examples of active pharmaceutical ingredients which can be used for the coatings include simvastatin, fenofibrate, pravastatin, propranolol, enalapril and prioglitazone.

Korean Patent Publication Nos. 2007-0038553, 2007-0108945 and 2009-0086078 disclose pharmaceutical compositions of directly mixing omega-3 fatty acid esters with statins, however their drug stability cannot be guaranteed when directly mixed. Also, Korean Patent Publication Nos. 2007-0108945 and 2007-0083715 relate to pharmaceutical compositions comprising statins or microcapsules thereif. The compositions are formulated into a soft capsule form, mixed with omega-3 fatty acid oils which cause a delayed release of statin, so the dissolution rate of statin is much slower than that of commercially available statin drugs.

Currently, there is no complex composition drug comprising omega-3 fatty acid esters and HMG-CoA reductase inhibitor available having the same dissolution rate of statin as the commercial statin drugs. Therefore, there has been a need to develop a complex composition comprising omega-3 fatty acid esters and HMG-CoA reductase inhibitors which is pharmaceutically stable; having the same dissolution rate and efficacy as commercial statin drugs; and does not show a delayed release behavior even after long-term storage.

### SUMMARY OF THE INVENTION

Accordingly, it is an object of the present invention to provide an oral complex composition comprising omega-3 fatty acid esters and rosuvastatin or a pharmaceutically acceptable salt thereof, which exhibits an improved rosuvastatin releasing rate and does not show a delayed release behavior even after long-term storage.

It is another object of the present invention to provide a method for preparing the oral complex composition.

In accordance with one aspect of the present invention, there is provided an oral complex composition, which comprises: (a) a soft capsule core comprising omega-3 fatty acid esters; (b) a first coating layer which encases the soft capsule core and comprises a hydrophobic coating material; and (c) a second coating layer deposited on the first coating layer, which comprises (i) rosuvastatin or a pharmaceutically acceptable salt thereof, and (ii) polyvinyl alcohol (PVA), polyvinyl alcohol-polyethylene glycol (PVA-PEG) graft copolymer, or a mixture thereof.

In accordance with another object of the present invention, there is provided a method for preparing the oral complex composition, which comprises the steps of: (1) preparing a soft capsule core comprising omega-3 fatty acid esters; (2) forming a first coating layer which encases the soft capsule core and comprises a hydrophobic coating material; and (3) forming a second coating layer on the first coating layer, which comprises (i) rosuvastatin or a pharmaceutically acceptable salt thereof, and (ii) PVA, PVA-PEG graft copolymer, or a mixture thereof.

### BRIEF DESCRIPTION OF THE DRAWINGS

The above and other objects and features of the present invention will become apparent from the following description of the invention, when taken in conjunction with the accompanying drawings, which respectively show:
Fig. 1: the disintegration times of the capsules obtained in Comparative Examples 1, 3 and Examples 1 to 7;
Fig. 2 : the disintegration times depending on the amount of ethyl cellulose in the capsules obtained in Comparative Example 3 and Examples 1 to 7;
Fig. 3 : the changes in water content of the capsules obtained in Comparative Examples 1, 3 and Examples 1 to 7; and
Fig. 4 : the changes in dissolution rates of rosuvastatin in the capsules obtained in Comparative Examples 2, 4, 5 and Examples 8 to 12.

### DETAILED DESCRIPTION OF THE INVENTION

The inventive oral complex composition is characterized by comprising: (a) a soft capsule core comprising omega-3 fatty acid esters; (b) a first coating layer which encases the soft capsule core and comprises a hydrophobic coating material; and (c) a second coating layer deposited on the first coating layer, which comprises the ingredients of (i) rosuvastatin or a pharmaceutically acceptable salt thereof, and (ii) PVA, PVA-PEG graft copolymer, or a mixture thereof.

Hereinafter, the components contained in the oral complex composition of the present invention are described in detail.

### (a) Soft capsule core

The soft capsule core of the complex composition according to the present invention comprises omega-3 fatty acid esters as the first active pharmaceutical ingredient.

In one embodiment of the present invention, the omega-3 fatty acid esters may comprise ethyl esters of EPA and DHA in an amount of 80% by weight or more, preferably at least 40% by weight of EPA ethyl ester of and at least 34% by weight of DHA ethyl ester. Preferably, the omega-3 fatty acid esters may comprise ethyl ester of omega-3 fatty acid in an amount of 90% by weight or more.

The amount of the omega-3 fatty acid esters present in the soft capsule core may be 100 mg to 2,000 mg.

In a preferred embodiment of the present invention, the amount of the omega-3 fatty acid esters may be 70% to 95% by weight, based on the total weight of the soft capsule core, but not limited thereto.

Also, the soft capsule core can be prepared in a conventional manner for manufacturing soft capsules by using typical materials for soft capsules, e.g. gelatins.

### (b) First coatining layer

In the oral complex composition of the present invention, the first coating layer comprises a hydrophobic coating material which encapsulates the soft capsule core in order to prevent a change in water content inside the soft capsule core from affecting the dissolution rate of rosuvastatin-containing second coating layer and increase of related materials. When rosuvastatin is coated directly on the soft capsule core containing omega-3 fatty acid ester, then the water content of the capsule can affect the content of the rosuvastatin, reduce its dissolution rate and increase its related materials. In the present invention, however, the first coating layer comprising a hydrophobic coating material is employed in between the omega-3 fatty acid ester-containing soft capsule core and the rosuvastatin-containing second coating layer, minimizing the effect of water content as well as other potential risks.

Examples of the hydrophobic coating material may include cellulose acetate, polyvinyl acetate, ethyl cellulose, and (meth)acrylic acid copolymers, i.e. Eudragit^{®}, preferably ethyl cellulose. The amount of the hydrophobic coating material used may be, based on the total amount of the first coating layer, 15% to 75% by weight, preferably 16% to 75% by weight, more preferably 16% to 72% by weight. If the amount of the hydrophobic coating material used is less than 15% by weight, the change in water content becomes greater, causing deterioration of storage stability by reducing rosuvastatin content and dissolution rate as well as increasing related materials.

The first coating layer may further comprise conventional coating materials such as hydroxypropyl methyl cellulose (HPMC) and hydroxylpropyl cellulose (HPC), which are generally used in the pharmaceutical field. In addition, the first coating layer may further comprise other pharmaceutically acceptable additives such as disintegrants, diluents, stabilizers, binders, and slip modifiers to the extent they do not adversely affect the disintegration rate of the capsule.

The first coating layer may be prepared by dissolving or dispersing the hydrophobic coating material in water, ethanol, or a mixture thereof, preferably in the mixed solvent, to obtain a coating solution and then applying the solution onto the surface of the soft capsule core.

The first coating layer may be coated on the soft capsule core in an amount of 2 parts by weight or more, preferably 4 to 10 parts by weight, based on 100 parts by weight of the soft capsule core.

### (c) Second coating layer

In the oral complex composition of the present invention, the second coating layer comprises the ingredients of: (i) rosuvastatin or a pharmaceutically acceptable salt thereof, as the second active pharmaceutical ingredient, and (ii) PVA, PVA-PEG graft copolymer or a mixture thereof, as a coating material, which permits rapid release of rosuvastatin when dissolved and also prevents delayed release of rosuvastatin after being stored.

The second coating layer may be prepared by dissolving or dispersing said ingredients (i) and (ii) in water, ethanol or a mixture thereof, preferably in the mixed solvent, to obtain a coating solution, followed by applying the solution onto the surface of the first coating layer.

The amount of the ingredient (i) employed in the second coating layer may be 1 mg to 50 mg, and the amount of the ingredient (ii) employed may be 25% to 85% by weight, preferably 25% to 80% by weight, based on the total amount of the second coating layer.

The second coating layer may further comprise HPMC, polyvinylpyrrolidone (PVP), or a mixture thereof, and may also comprise other pharmaceutically acceptable additives such as alkaline stabilizers, if necessary.

The second coating layer may be coated on the first coating layer in an amount of 3 to 30 parts by weight, preferably 5 to 20 parts by weight, based on 100 parts by weight of the soft capsule core.

The present invention also provides a method for preparing the oral complex composition of the present invention, which comprises the steps of: (1) preparing a soft capsule core comprising omega-3 fatty acid esters; (2) forming a first coating layer which encases the soft capsule core and comprises a hydrophobic coating material; and (3) forming a second coating layer on the first coating layer, which comprises the ingredients of (i) rosuvastatin or a pharmaceutically acceptable salt thereof, and (ii) PVA, PVA-PEG graft copolymer, or a mixture thereof.

Specifically, the method for preparing the oral complex composition of the present invention may comprise the following steps of: (1) preparing a soft capsule core containing omega-3 fatty acid esters in a conventional manner for manufacturing soft capsules; (2) forming a first coating layer encasing the soft capsule core by dissolving a hydrophobic coating material in a suitable solvent for barrier coating, e.g. in a mixture of ethanol and water, applying the coating solution onto the soft capsule core, and then drying the solution; and (3) forming a second coating layer on the first coating layer by dissolving the ingredients (i) and (ii) with alkaline stabilizers and/or other pharmaceutically acceptable additives in a suitable solvent, e.g. in a mixture of ethanol and water, applying the coating solution onto the surface of the first coating layer, and then drying the solution.

The complex composition of the present invention can be formulated into a capsule form, and can be administered orally.

The oral complex composition of the present invention can show a rosuvastatin or its pharmaceutically acceptable salt releasing rate of 80% or more in 0.05M citrate buffer within 30 min, preferably within 10 min; and no delayed release after storage duration of 6 months at 40°C under 75% relative humidity (RH). The above test results indicate good dissolution rate and storage stability and hence, it is possible to use in composite forms.

The oral complex composition of the present invention contains both omega-3 fatty acid esters and rosuvastatin, as active pharmaceutical ingredients, which can raise serum HDL level while reducing both LDL and TG levels. Thus, it can be used for effectively preventing or treating hypertriglyceridemia, hypercholesteremia, coronary arterial heart diseases (CAHD), dyslipidemia, and increased serum LDL cholesterol levels, and it can be used for preventing or treating cardiovascular diseases as well.

### EXAMPLES

The following Examples are provided to illustrate preferred embodiments of the present invention, and are not intended to limit the scope of the present invention.

### Preparation of soft capsule core

### Comparative Example 1: Soft capsule comprising omega-3 fatty acid ester

A soft capsule was prepared with 1,000 mg of omega-3 fatty acid ester oils by using a conventional method for manufacturing soft gelatin capsule.

### Comparative Example 2: Soft capsule comprising omega-3 fatty acid esters and rosuvastatin

A soft capsule was prepared by repeating the procedures of Comparative Example 1, except for employing a homogeneous mixture of 1,000 mg of omega-3 fatty acid ester oils and 10 mg of rosuvastatin.

### First coating

### Comparative Example 3: Omega-3 fatty acid ester soft capsule with conventional barrier coating

In accordance with the ingredients described in Table 1, hydroxypropyl methyl cellulose (HPMC 2910, Shinetsu Co. Ltd., Japan), polyethylene glycol (PEG 6000, Sanyo Chemical Industries, Ltd., Japan) and polyvinylpyrrolidone (PVP K-30, BASF SE, Germany) were added to a mixture of ethanol and water, and then mixed to obtain a coating solution.

The coating solution was coated onto the surface of the soft capsule core prepared in Comparative Example 1 by using a coater (SFC-30, SEJONG Co., Ltd.), wherein the supply air temperature was 45°C and the product temperature was adjusted to 30°C. The product was dried for 30 minutes to remove residual solvent so that a soft capsule with a barrier coating was obtained.

### Examples 1 to 7: Omega-3 fatty acid ester soft capsule with hydrophobic barrier coating

In accordance with the ingredients described in Table 1, a soft capsule with a hydrophobic barrier coating was prepared by repeating the procedures of Comparative Example 3, except for adding ethyl cellulose (Dow Chemical Company, US) as a hydrophobic coating material.

The compositions of soft capsules and barrier coatings are summarized in Table 1.

**Table 1**

| | **Comp. Ex. 3** | **Ex. 1** | **Ex. 2** | **Ex. 3** | **Ex. 4** | **Ex. 5** | **Ex. 6** | **Ex. 7** |
|---|---|---|---|---|---|---|---|---|
| **Soft capsule core** | Comp. Ex. 1 | Comp. Ex. 1 | Comp. Ex. 1 | Comp. Ex. 1 | Comp. Ex. 1 | Comp. Ex. 1 | Comp. Ex. 1 | Comp. Ex. 1 |
| **HPMC 2910** (% by weight) | 80 | 72 | 64 | 48 | 32 | 16 | 8 | 0 |
| **PEG 6000** (% by weight) | 10 | 10 | 10 | 10 | 10 | 10 | 10 | 10 |
| **PVP K-30** (% by weight) | 10 | 10 | 10 | 10 | 10 | 10 | 10 | 10 |
| **Ethyl cellulose** (% by weight) | 0 | 8 | 16 | 32 | 48 | 64 | 72 | 80 |
| **Ethanol** (mg) | (1040) | (1040) | (1040) | (1040) | (1040) | (1040) | (1040) | (1040) |
| **Distilled water** (mg) | (440) | (440) | (440) | (440) | (440) | (440) | (440) | (440) |
| **Total amount of first coating layer** (% by weight) | 100 | 100 | 100 | 100 | 100 | 100 | 100 | 100 |
| **EC content in first coating layer** (% by weight) | 0 | 8.0 | 16.0 | 32.0 | 48.0 | 64.0 | 72.0 | 80.0 |

### Second coating

### Comparative Examples 4 to 6: Omega-3 fatty acid ester soft capsule with rosuvastatin coating

Rosuvastatin calcium salt, hydroxypropyl methylcellulose (HPMC 2910, Shinetsu Co., Ltd., Japan), polyethylene glycol (PEG 6000, Sanyo Chemical Industries, Ltd., Japan) and PVA (Kurary Co., Ltd., Japan) were added to a mixture of ethanol and water, and then mixed to obtain a coating solution.

The coating solution was coated onto the surface of the omega-3 fatty acid ester soft capsules obtained in Comparative Examples 1, 3 and Example 5, respectively, by using a coater (SFC-3, SEJONG Co., Ltd.), wherein the supply air temperature was 45°C and the product temperature was adjusted to 30°C. The products were dried for 30 minutes to remove residual solvent so that complex compositions were obtained.

### Examples 8 to 12: Omega-3 fatty acid ester soft capsule with rosuvastatin coating

Rosuvastatin calcium, hydroxypropyl methylcellulose (HPMC 2910, Shinetsu Co., Ltd., Japan), and polyethylene glycol (PEG 6000, Sanyo Chemical Industries, Ltd., Japan) were added to a mixture of ethanol and water. Further, PVA (Kurary Co., Ltd., Japan) or PVA-PEG graft copolymer (Kollicoat IR, BASF SE, Germany) was added thereto and then mixed to obtain a coating solution.

The coating solution was coated onto the surface of the omega-3 fatty acid ester soft capsule obtained in Example 5 by using a coater (SFC-3, SEJONG Co., Ltd.), wherein the supply air temperature was 45°C and the product temperature was adjusted to 30°C. The products were dried for 30 minutes to remove residual solvent so that complex compositions were obtained.

The compositions of second coating layers are summarized in Table 2.

**Table 2**

| | **Comp. Ex.4** | **Comp. Ex. 5** | **Comp. Ex.6** | **Ex. 8** | **Ex. 9** | **Ex. 10** | **Ex. 11** | **Ex. 12** |
|---|---|---|---|---|---|---|---|---|
| **Soft capsule core (with first coating)** | Comp. Ex. 1 | Comp. Ex. 3 | Ex. 5 | Ex. 5 | Ex. 5 | Ex. 5 | Ex. 5 | Ex. 5 |
| **Rosuvastatin calcium** (mg) | 10.4 | 10.4 | 10.4 | 10.4 | 10.4 | 10.4 | 10.4 | 10.4 |
| **HPMC 2910(P645)** (mg) | 10 | 10 | 54 | 36 | 26 | 10 | 0 | 0 |
| **PEG 6000** (mg) | 5.6 | 5.6 | 5.6 | 5.6 | 5.6 | 5.6 | 5.6 | 5.6 |
| **PVA** (mg) | 44 | 44 | 0 | 18 | 28 | 44 | 54 | 0 |
| **Kollicoat IR** (mg) | 0 | 0 | 0 | 0 | 0 | 0 | 0 | 54 |
| **Ethanol** (mg) | (644) | (644) | (644) | (644) | (644) | (644) | (460) | (460) |
| **Distilled water** (mg) | (276) | (276) | (276) | (276) | (276) | (276) | (460) | (460) |
| **Total weight of second coating layer** (mg) | 70 | 70 | 70 | 70 | 70 | 70 | 70 | 70 |
| **PVA or Kollicoat content in second coating layer** (% by weight) | 62.9 | 62.9 | 0.0 | 25.7 | 40.0 | 62.9 | 77.1 | 77.1 |
| **PVA/(HPMC+PVA)** (% by weight) | 81.5 | 81.5 | 0.0 | 33.3 | 51.9 | 81.5 | 100.0 | 100.0 |

### EVALUATION

### Test 1: Disintegration test of soft capsules with barrier coating

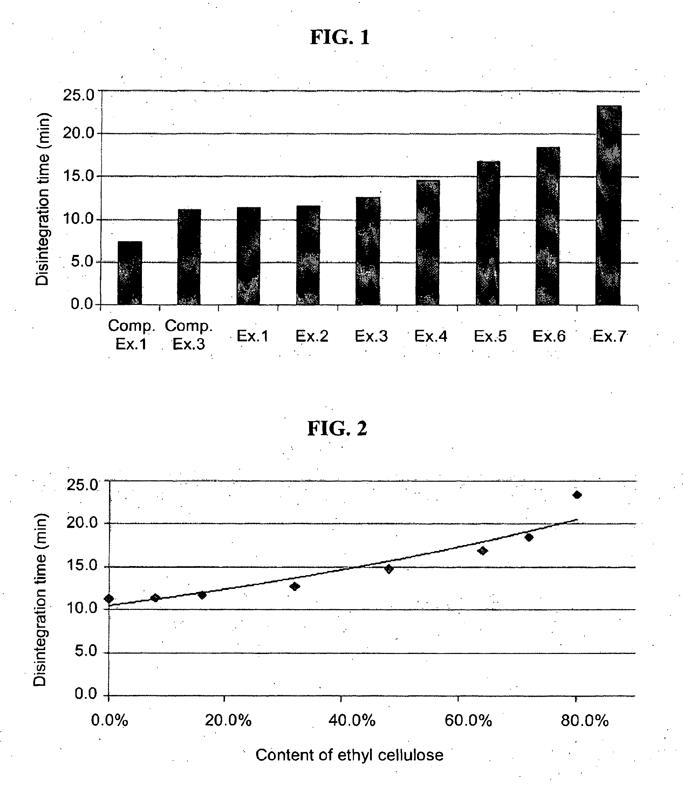

A disintegration test was performed on the soft capsule prepared in Comparative Example 1, the soft capsule with barrier coating prepared in Comparative Example 3 and the soft capsules with hydrophobic coating prepared in Examples 1 to 7, according to the method described in the general test method of the Korean Pharmacopoeia. The results are shown in Figs. 1 and 2.

As shown in Figs. 1 and 2, the soft capsule prepared in Comparative Examples 1 and 3 showed satisfying results in disintegration time of less than 20 minutes according to the standards of the Korean Pharmacopoeia.

Satisfactory results were also obtained from the soft capsules prepared in Examples 1 to 7. The soft capsule prepared in Example 7, however, showed a delay in disintegration time due to added hydrophobic excipient, i.e. ethyl cellulose, in an amount exceeded 75% by weigh for preventing a change in water content.

Therefore, it can be found that the preferable amount of the hydrophobic coating material in the first coating layer is 75% by weight or less, more preferably 72% by weight or less, based on the weight of the first coating layer, in order to satisfy the standards of the disintegration test.

### Test 2: Water absorption rate of soft capsules with barrier coating

The capsules prepared in Comparative Examples 1 and 3, and Examples 1 to 7 were dried for 6 days at 30% RH, and the water content of gelatin capsule coating was measured. The water content change was observed by measuring weight loss on drying according to the method as described in the general test method of the Korean Pharmacopoeia, wherein the measurement was taken at 90°C in an equilibrium state, where weight of the sample no longer changes, and then the results were recorded as the initial water contents.

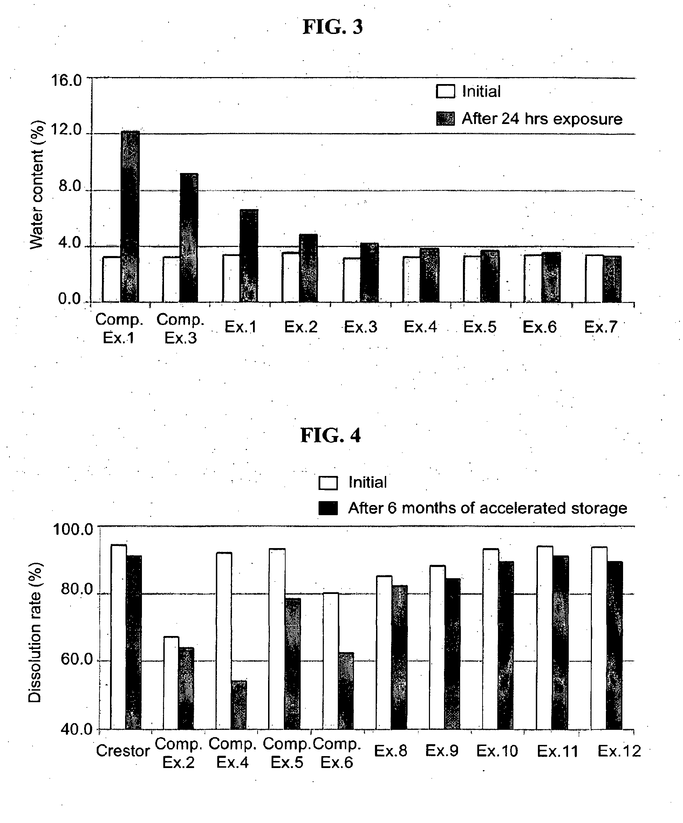

The samples were also tested for water penetration prevention effect by repeating the measuring method after 24 hrs exposure at 25 °C/60% RH and the results were recorded as the final water content, which are shown in Fig. 3.

As shown in Fig. 3, the soft capsules with no coating and conventional barrier coating as prepared in Comparative Example 1 and 3, respectively, showed a relatively great change in water content, i.e., 6% or more, as compared with the initial water content.

However, the soft capsules with hydrophobic coating containing ethyl cellulose prepared in Examples 1 to 7 resulted a slight change in water content. In particular, the soft capsules with hydrophobic coating containing ethyl cellulose in an amount of 16% or more, resulted only 3% change or less in water content, which shows the employment of ethyl cellulose significantly improves stability of the composition by reducing the water absorption by 50% or greater in comparison to coatings without ethyl cellulose. Further, it can be found that the preferable amount of the hydrophobic coating material used in the first coating layer is 15% to 75% by weight, more preferably 16% to 75% by weight, most preferably 16% to 72% by weight, based on the weight of the first coating layer.

### Test 3: Dissolution rate of rosuvastatin

A dissolution test of rosuvastatin was performed on Crestor^{®} (AstraZeneca plc, UK) as a control drug, and the complex compositions prepared in Comparative Examples 2, 4 to 6, and Examples 8 to 12.

The test was conducted, based on paddle method in the Korean Pharmacopoeia, at 50 rpm by using dissolution medium of 900 mL of 0.05 M citric acid buffer and the mediums were collected after 10 minutes, then their initial dissolution rates were measured. Also, the samples were stored in sealed HDPE bottles for six months under accelerated storage condition (40°C/75% RH) and their dissolution rates were measured. The results are shown in Fig. 4.

As shown in Fig. 4, the complex composition prepared in Comparative Example 2 had poor dissolution rate of rosuvastatin from the initial measurement. The complex compositions prepared in Comparative Examples 4 and 5 had decent initial dissolution rates of 80% or greater, but the rates deteriorated after six months of accelerated storage. Also, the complex composition prepared in Comparative Example 6, which did not contain any PVA or PVA-PEG graft copolymer, had poor dissolution rate in comparison to the complex compositions of Examples 8 to 12.

On the other hand, the complex compositions prepared in Examples 8 to 12, wherein the first coating layer contains hydrophobic coating material, i.e., ethyl cellulose, in an amount of 16% to 72% by weight and the second coating layer contains PVA or PVA-PEG graft copolymer with rosuvastatin, had initial dissolution rates of 80% or greater. Also, the complex compositions exhibited little or no delayed release even after 6 months of accelerated storage, showing pharmaceutically stable dissolution rates. Accordingly, it can be found from the results that the complex composition of the present invention has *in vivo* efficacy.

While the invention has been described with respect to the above specific embodiments, it should be recognized that various modifications and changes may be made to the invention by those skilled in the art which also fall within the scope of the invention as defined by the appended claims.

## Claims

1. An oral complex composition, which comprises:
(a) a soft capsule core comprising omega-3 fatty acid esters;
(b) a first coating layer which encases the soft capsule core and comprises a hydrophobic coating material; and
(c) a second coating layer deposited on the first coating layer, which comprises:
(i) rosuvastatin or a pharmaceutically acceptable salt thereof, and
(ii) polyvinyl alcohol, polyvinyl alcohol-polyethylene glycol graft copolymer or a mixture thereof.

2. The oral complex composition of claim 1, wherein the omega-3 fatty acid esters comprise ethyl esters of eicosapentaenoic acid and docosahexaenoic acid in an amount of 80% by weight or more.

3. The oral complex composition of claim 1, wherein the omega-3 fatty acid esters are present in an amount ranging from 100 mg to 2,000 mg.

4. The oral complex composition of claim 1, wherein the hydrophobic coating material is selected from the group consisting of cellulose acetate, polyvinyl acetate, ethyl cellulose, (meth)acrylic acid copolymers and a mixture thereof.

5. The oral complex composition of claim 1, wherein the hydrophobic coating material is present in an amount ranging from 15% to 75% by weight based on the total amount of the first coating layer.

6. The oral complex composition of claim 1, wherein rosuvastatin or the pharmaceutically acceptable salt thereof is present in an amount ranging from 1 mg to 50 mg.

7. The oral complex composition of claim 1, wherein polyvinyl alcohol, polyvinyl alcohol-polyethylene glycol graft copolymer or the mixture thereof is present in an amount ranging from 25% to 85% by weight based on the total amount of the second coating layer.

8. The oral complex composition of claim 1, which shows a rosuvastatin or its pharmaceutically acceptable salt releasing rate of 80% or more in 0.05 M citrate buffer within 10 min.

9. A method for preparing the oral complex composition of claim 1, which comprises the steps of:
(1) preparing a soft capsule core comprising omega-3 fatty acid esters;
(2) forming a first coating layer which encases the soft capsule core and comprises a hydrophobic coating material; and
(3) forming a second coating layer on the first coating layer, which comprises:
(i) rosuvastatin or a pharmaceutically acceptable salt thereof, and
(ii) polyvinyl alcohol, polyvinyl alcohol-polyethylene glycol graft copolymer or a mixture thereof.

## Patentansprüche

1. Komplexe orale Zusammensetzung, welche umfasst:
(a) einen Weichkapselkern umfassend Omega-3-Fettsäureester;
(b) eine erste Beschichtungsschicht, die den Weichkapselkern umhüllt und ein hydrophobes Beschichtungsmaterial umfasst; und
(c) eine zweite Beschichtungsschicht, die auf der ersten Beschichtungsschicht aufgetragen ist, welche umfasst:
(i) Rosuvastatin oder ein pharmazeutisch annehmbares Salz desselben, und
(ii) Polyvinylalkohol, Polyvinylalkohol-Polyethylenglykol-Pfropfcopolymer oder eine Mischung derselben.

2. Komplexe orale Zusammensetzung nach Anspruch 1, wobei die Omega-3-Fettsäureester Ethylester von Eicosapentaensäure und Docosahexaensäure in einer Menge von 80 Gew.-% oder mehr umfassen.

3. Komplexe orale Zusammensetzung nach Anspruch 1, wobei die Omega-3-Fettsäureester in einer Menge im Bereich von 100 mg bis 2.000 mg vorhanden sind.

4. Komplexe orale Zusammensetzung nach Anspruch 1, wobei das hydrophobe Beschichtungsmaterial ausgewählt ist aus der Gruppe bestehend aus Celluloseacetat, Polyvinylacetat, Ethylcellulose, (Meth)acrylsäure-Copolymeren und einer Mischung derselben.

5. Komplexe orale Zusammensetzung nach Anspruch 1, wobei das hydrophobe Beschichtungsmaterial in einer Menge im Bereich von 15 bis 75 Gew.-% basierend auf der Gesamtmenge der ersten Beschichtungsschicht vorhanden ist.

6. Komplexe orale Zusammensetzung nach Anspruch 1, wobei Rosuvastatin oder das pharmazeutisch annehmbare Salz desselben in einer Menge im Bereich von 1 mg bis 50 mg vorhanden ist.

7. Komplexe orale Zusammensetzung nach Anspruch 1, wobei Polyvinylalkohol, Polyvinylalkohol-Polyethylenglykol-Pfropfcopolymer oder die Mischung derselben in einer Menge im Bereich von 25 bis 85 Gew.-% basierend auf der Gesamtmenge der zweiten Beschichtungsschicht vorhanden sind.

8. Komplexe orale Zusammensetzung nach Anspruch 1, welche eine Freisetzungsrate von Rosuvastatin oder seinem pharmazeutisch annehmbaren Salz von 80% oder mehr in 0,05 M Citratpuffer innerhalb von 10 Minuten zeigt.

9. Verfahren zum Herstellen der komplexen oralen Zusammensetzung nach Anspruch 1, welches die Schritte umfasst:
(1) Herstellen eines Weichkapselkerns umfassend Omega-3-Fettsäureester;
(2) Bilden einer ersten Beschichtungsschicht, die den Weichkapselkern umhüllt und ein hydrophobes Beschichtungsmaterial umfasst; und
(3) Bilden einer zweiten Beschichtungsschicht auf der ersten Beschichtungsschicht, welche umfasst:
(i) Rosuvastatin oder ein pharmazeutisch annehmbares Salz desselben, und
(ii) Polyvinylalkohol, Polyvinylalkohol-Polyethylenglykol-Pfropfcopolymer oder eine Mischung derselben.

## Revendications

1. Composition complexe administrable par voie orale contenant :
(a) un noyau de capsule de matière molle contenant des esters d'acides gras oméga-3
(b) une première couche d'enrobage qui revêt le noyau de capsule de matière molle et qui renferme un matériau d'enrobage hydrophobe ; et
(c) une seconde couche d'enrobage déposée sur la première couche d'enrobage qui comprend :
(i) de la rosuvastatine ou un sel acceptable sur le plan pharmaceutique de celle-ci, et
(ii) de l'alcool polyvinylique, un polymère greffé d'alcool polyvinylique-polyéthylène glycol ou un mélange de ceux-ci.

2. Composition complexe administrable par voie orale selon la revendication 1, dans laquelle les esters d'acides gras oméga-3 comprennent des esters d'éthyle de l'acide eicosapentaénoïque et de l'acide docosahexaénoïque en une quantité de 80 % en poids ou supérieure.

3. Composition complexe administrable par voie orale selon la revendication 1, dans laquelle les esters d'acides gras oméga-3 sont présents en une quantité comprise entre 100 mg et 2.000 mg.

4. Composition complexe administrable par voie orale selon la revendication 1, dans laquelle le matériau d'enrobage hydrophobe est sélectionné dans le groupe constitué d'acétate de cellulose, d'acétate de polyvinyle, de cellulose d'éthyle, de copolymères d'acide (méth)acrylique et d'un mélange de ceux-ci.

5. Composition complexe administrable par voie orale selon la revendication 1, dans laquelle le matériau d'enrobage hydrophobe est présent en une quantité comprise entre 15 % et 75 % en poids par rapport à la quantité totale de la première couche d'enrobage.

6. Composition complexe administrable par voie orale selon la revendication 1, dans laquelle la rosuvastatine ou le sel acceptable sur le plan pharmaceutique de celle-ci est présent en une quantité comprise entre 1 mg et 50 mg.

7. Composition complexe administrable par voie orale selon la revendication 1, dans laquelle l'alcool polyvinylique, le copolymère greffé d'alcool polyvinylique-polyéthylène glycol ou le mélange de ceux-ci est présent en une quantité comprise entre 25 % et 85 % en poids par rapport à la quantité totale de la seconde couche d'enrobage.

8. Composition complexe administrable par voie orale selon la revendication 1, qui présente une vitesse de libération de la rosuvastatine ou de son sel acceptable sur le plan pharmaceutique de 80 % ou supérieure dans un tampon citrate d'une molarité de 0,05 M en 10 minutes.

9. Procédé de préparation de la composition complexe administrable par voie orale selon la revendication 1, qui comprend les étapes consistant à :
(1) préparer un noyau de capsule de matière molle contenant des esters d'acides gras oméga-3
(2) former une première couche d'enrobage qui revêt le noyau de capsule de matière molle et qui renferme un matériau d'enrobage hydrophobe ; et
(3) former une seconde couche d'enrobage déposée sur la première couche d'enrobage qui comprend :
(i) de la rosuvastatine ou un sel acceptable sur le plan pharmaceutique de celle-ci, et
(ii) de l'alcool polyvinylique, un polymère greffé d'alcool polyvinylique-polyéthylène glycol ou un mélange de ceux-ci.
